(19) Europäisches Patentamt
European Patent Office
Office européen des brevets

(11) Numéro de publication: **0 173 597**
**B1**

(12) **FASCICULE DE BREVET EUROPÉEN**

(45) Date de publication du fascicule du brevet: **27.12.89**

(51) Int. Cl.⁴: **C 07 D 233/22,** A 61 K 31/415

(21) Numéro de dépôt: **85401357.0**

(22) Date de dépôt: **04.07.85**

(54) **Nouveaux éther-oxydes dérivés de cyclopropylphénols.**

(30) Priorité: **13.07.84 FR 8411271**

(43) Date de publication de la demande:
**05.03.86 Bulletin 86/10**

(45) Mention de la délivrance du brevet:
**27.12.89 Bulletin 89/52**

(84) Etats contractants désignés:
**AT BE CH DE FR GB IT LI LU NL SE**

(56) Documents cité:
**FR-A-2 394 531**

**CHEMICAL ABSTRACTS, vol. 100, no. 13, 26 mars 1984, page 19, no. 96148m, Columbus, Ohio, US; K. DHARMSATHAPHORN et al.: "Effects of structure-activity relationships of alpha-adrenergic compounds on electrolyte transport in the rabbit ileum and rat colon", & GASTROENTEROLOGY 1984, 86(1), 120-8**

(73) Titulaire: **Najer, Henri, 2 avenue Emile Acollas, F-75007 Paris (FR)**
Titulaire: **Giudicelli, François, 17 rue du Clos d'Orléans, F-94120 Fontenay- sous- Bois (FR)**
Titulaire: **Dufour, Jean- Claude, 4 Rond- Point Saint- James, F-92200 Neuilly- sur- Seine (FR)**

(72) Inventeur: **Najer, Henri, 2 avenue Emile Acollas, F-75007 Paris (FR)**
Inventeur: **Giudicelli, François, 17 rue du Clos d'Orléans, F-94120 Fontenay- sous- Bois (FR)**
Inventeur: **Dufour, Jean- Claude, 4 Rond- Point Saint- James, F-92200 Neuilly- sur- Seine (FR)**

(74) Mandataire: **Combe, André, CABINET BEAU DE LOMENIE 55, rue d'Amsterdam, F-75008 Paris (FR)**

**EP 0 173 597 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention a pour objet de nouvelles imidazolines de formule générale (I) :

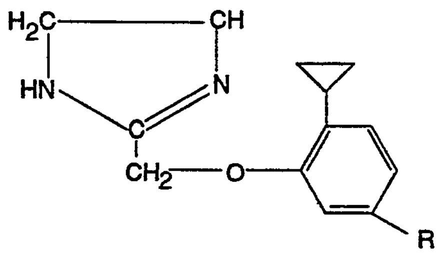

(I)

ainsi que les sels d'addition formés par ces bases avec les acides minéraux ou organiques pharmaceutiquement acceptables, parmi lesquels certains, comme les acides chlorhydrique, sulfurique nitrique, acétique, tartrique et citrique sont dénués de propriétés pharmacologiques propres, tandis que d'autres sont porteurs d'une activité thérapeutique complémentaire à celle des composés (I) tels les acides suivants : acide prednisolone benzoylmétasulfonique-21; acide fluoro-9α méthyl-16ß trihydroxy-11ß,17α,21 prégnadiène-1,4 dione-3,20 orthophosphorique-21; acide trihydroxy-11ß,17α,21 prégnadiène-1,4 dione-3,20 orthophosphorique-21; acide trihydroxy-11ß,17α,21 prégnène-4 dione-3,20 orthophosphorique-21; acide trihydroxy11ß,17α,21 prégnène-4 dione-3,20 succinique-21.

Font également partie de la présente invention le procédé de préparation des imidazolines (I), leur utilisation à titre de médicaments nouveaux et les compositions médicamenteuses les renfermant.

L'invention concerne donc les composés de formule générale (I) dans lesquels R représente un radical méthyle ou un atome de chlore.

Lesdits composés sont donc : celui pour lequel R est un reste méthyle, à savoir la (méthyl-5' cyclopropyl-2')phényloxyméthyl-2$\Delta_2$-imidazoline et celui pour lequel R est un atome de chlore, à savoir le (chloro-5' cyclopropyl-2')phényloxyméthyl-2$\Delta_2$-imidazoline, ils ont montré des propriétés tout à fait remarquables et inattendues.

Le brevet français 2 145 423 décrit la (cyclopropyl-2')-phényloxyméthyl-$\Delta 2_2$-imidazoline, connue depuis sous la dénomination commune "Cirazoline", comme un puissant vaso-constricteur nasal. Cette activité vaso-constrictrice est en rapport avec une action agoniste au niveau des récepteurs $\alpha_1$-adrénergiques postsynaptiques.

Le brevet français 2 394 531 enseigne également que certains dérivés de la phénoxy-méthyl-2-imidazoline possèdent des propriétés vaso-constrictrices.

D'une façon tout à fait surprenante, les imidazolines (I), pour lesquelles R = $CH_3$ ou C1, exercent des activités vaso-constrictrices supérieures et plus spécifiques sur les récepteurs $\alpha_1$-adrénergiques postsynaptiques que la Cirazoline.

Par contre, au niveau présynaptique, ni les imidazolines (I), ni d'ailleurs la Cirazoline, n'exercent des effets agonistes $\alpha_2$ ou antagonistes $\alpha_2$ adrénergiques.

La présente invention concerne donc également les médicaments contenant comme produit actif au moins un composé de formule générale (I). Lesdits médicaments sont plus particulièrement utiles comme vaso-constricteurs et décongestionnants et sont actifs sur les muqueuses nasales. Lorsque les sels sont réalisés avec des acides présentant une activité thérapeutique propre, il se trouve que le produit obtenu possède les propriétés des deux constituants desdits sels et souvent des propriétés supérieures pour le traitement des syndromes rhinologiques.

L'invention a également pour objet le procédé de préparation des dérivés (I) suivant le schéma (A) ou ses variantes, par exemple (B) :

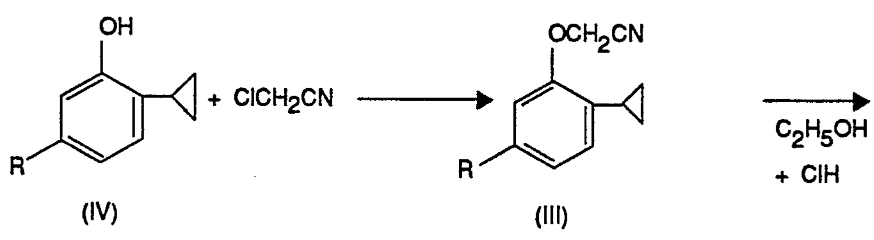

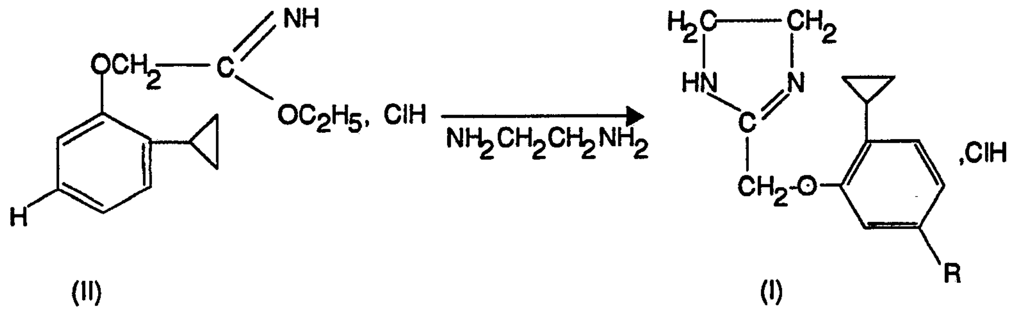


(II) (I)

Schéma (A)

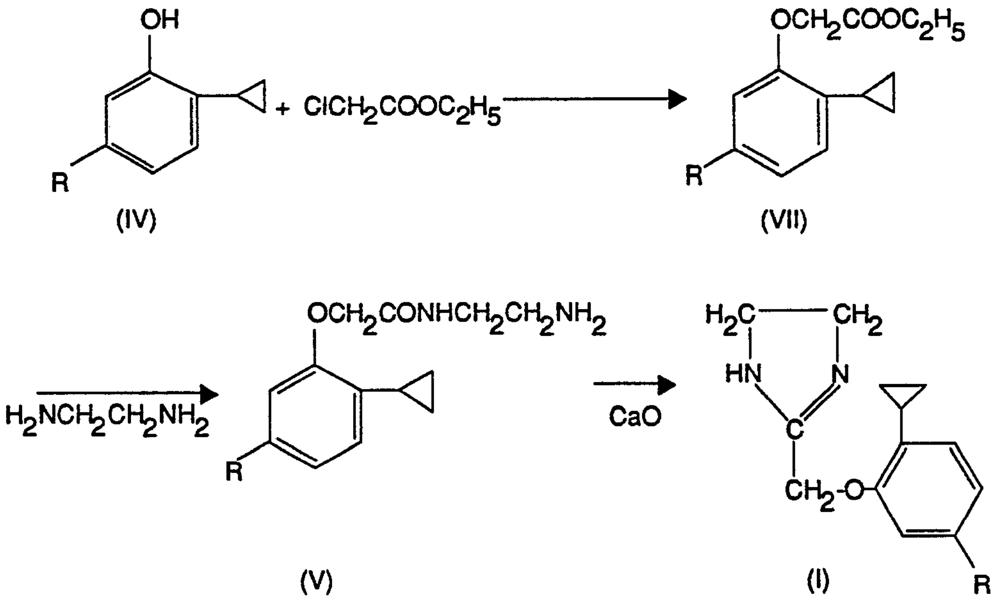


(IV) (VII)

(V) (I)

**Variante (B)**

R ayant les significations précédemment définies.

Le procédé suivant le schéma (A) consiste à faire réagir l'éthylène diamine avec un imino-éther de formule générale (II). La réaction s'effectue en général au sein d'un alcool de faible poids moléculaire, de préférence l'alcool éthylique, et à la température du reflux de ce solvant, la durée de ce chauffage étant de quelques heures, de préférence quatre à six heures. L'imino-éther (II) est préparé en condensant un nitrile de formule générale (III) avec une quantité équimoléculaire d'un alcool, de préférence l'alcool éthylique, au sein d'un solvant inerte, de préférence l'éther, et en faisant passer dans cette solution un courant d'acide chlorhydrique jusqu'à saturation. Cette condensation s'opère à basse température, de préférence entre 0 et 10° C et en abandonnant le mélange pendant douze à vingt-quatre heures. On récupère le chlorhydrate d'imino-éther (II) soit en essorant le précipité formé, soit en évaporant le solvant sous vide à basse température.

On prépare les nitriles (III) en chauffant une molécule d'un phénol de formule générale (IV) et 1,5 molécule de chloracétonitrile au sein d'un solvant inerte, de préférence la méthyléthylcétone et en présence d'un accepteur d'acide chlorhydrique, de préférence un carbonate alcalin tel le carbonate de potassium. La température de chauffage est celle du reflux du solvant et sa durée de quelques heures, de préférence une dizaine d'heures.

Le procédé suivant la variante (B) consiste à chauffer une amido-amine de formule générale (V) à une température de 150 à 200°C, en présence de chaux vive.

On prépare les amido-amines (V) en condensant un ester de formule générale (VI) avec un large excès d'éthylène diamine à 100°, la durée de chauffage étant de quelques heures, de préférence dix heures à vingt heures.

On prépare les esters (VII) en chauffant une molécule d'un phénol (IV) et 1,5 molécule du chloroacétate

d'éthyle au sein d'un solvant inerte, de préférence la méthyléthylcétone, et en présence d'un accepteur d'acide chlorhydrique, de préférence un carbonate alcalin tel le carbonate de potassium. La température de chauffage est celle du reflux du solvant employé et sa durée de quelques heures, de préférence une vingtaine d'heures.

Les phénols de formule générale (IV) en tant qu'intermédiaires dans la préparation des imidazolines (I) sont obtenus à partir des anilines substituées de formule générale (VIII) :

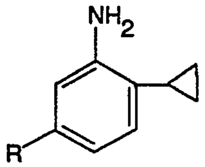

(VIII)

dans laquelle R a la même signification que précédemment, par traitement au nitrite de sodium en milieu acide, suivi de chauffage et entraînement à la vapeur.

Les sels d'addition des imidazolines (I) faisant l'objet de la présente invention sont obtenus par tous procédés connus de préparation des sels d'addition.

Pour expliciter la préparation des produits selon l'invention, un certain nombre d'exemples de réalisation sont donnés ci-après, sans toutefois que les précisions apportées, aussi bien en ce qui concerne les détails opératoires tels que température, durée de réaction, nature du solvant, que les proportions relatives des réactants, puissent être considérées comme ayant un caractère limitatif.

**Exemple 1**

**Chlorhydrate de la (méthyl-5' cyclopropyl-2')phenyloxyméthyl-2$\Delta_2$-imidazoline (I) R = $CH_3$** (N.D. 83 201)

*a) Méthyl-5 cyclopropyl-2 phénol :*

Dans un ballon de 500 $cm^3$ à 3 tubulures muni d'une ampoule à brome, d'un thermomètre, d'un agitateur mécanique et d'un réfrigérant à reflux, on introduit 73,5 $cm^3$ d'eau et 10,5 $cm^3$ d'acide sulfurique concentré. Par l'ampoule à brome, on ajoutte goutte à goutte, en environ 15 min, 17,2 g (0,117 mol) de méthyl-5 cyclopropyl-2 aniline. On porte ce mélange à la température de 60°C pour dissoudre le sel formé. On refroidit ensuite la solution à 0°C en l'agitant vigoureusement, puis on additionne par l'ampoule à brome, en 1 h, une solution de 7,8 g (0,117 mol/g) de nitrite de sodium dans 10,5 $cm^3$ d'eau glacée. On laisse la température du mélange réactionnel remonter à la température ambiante, puis le porte à 50°C - 60°C pendant 30 minutes (fin du dégagement des vapeurs nitreuses). On le laisse refroidir et on entraîne le phénol formé à la vapeur.

On extrait le distillat deux fois successivement par 200 $cm^3$ de chlorure de méthylène, on sèche les extraits réunis sur du sulfate de sodium sec, on filtre, on évapore le solvant et on rectifie le résidu.

On recueille finalement 8,1 g (rendement : 58 %) de méthyl-5 cyclopropyl-2 phénol, passant à 64 - 70°C sous 0,1 mm de mercure.

*b) Méthyl-5 cyclopropyl-2 phénoxyacétonitrile*

Dans un ballon de 250 $cm^3$ à 3 tubulures muni d'un agitateur mécanique, d'un thermomètre plongeant, d'une ampoule à brome et d'un réfrigérant surmonté d'un tube de garde rempli de chlorure de calcium, on introduit 13,2 g (0,0891 mol/g) de méthyl-5 cyclopropyl-2 phénol, 90 $cm^3$ de méthyléthylcétone séchée sur siliporite et 13,8 g (0,1 mol/g) de carbonate de potassium. On ajoute à ce mélange, par l'ampoule à brome, 9,1 ml de chloroacétonitrile, on le porte pendant 7 h à la température du reflux. On l'abandonne pendant une nuit à la température ambiante, filtre un insoluble, lave ce dernier par 100 $cm^3$ de méthyléthylcétone et évapore ce dernier du filtrat. On ajoute au résidu 100 $cm^3$ d'éther anhydre séché sur siliporite, agite vigoureusement, filtre un insoluble que l'on lave par 70 $cm^3$ d'éther anhydre, réunit les phases éthérées, les lave par deux fois successivement 70 $cm^3$ de soude normale, réextrait les phases aqueuses par quatre fois successivement 50 $cm^3$ d'éther, lave les phases éthérées réunies par quatre fois 50 $cm^3$ d'eau, les sèche sur du sulfate de sodium, filtre, évapore le solvant et rectifie le résidu.

On recueille finalement 11 g (rendement : 66,5 %) de méthyl-5 cyclopropyl-2 phénoxyacétonitrile passant à 113°C - 117°C sous 0,5 mm de mercure.

*c) Chlorhydrate de l'imino-éther éthylique de l'acide (méthyl-5 cyclopropyl-2 phénoxy)acétique :*

Dans un ballon de 250 $cm^3$ à 3 tubulures muni d'un réfrigérant surmonté d'un tube de garde rempli de chlorure de calcium, d'un thermomètre plongeant d'un agitateur et d'un chandelier, on introduit sous atmosphère d'azote, 8,2 g

(0,045 mol/g) de méthyl-5 cyclopropyl-2 phénoxyacétonitrile, 2,8 $cm^3$ d'éthanol anhydre et 50 cm d'éther séché sur du siliporite. On refroidit le mélange à 0°C et on y fait barboter un courant d'acide chlorhydrique jusqu'à saturation (environ 7 h). On abandonne ensuite le mélange réactionnel pendant une nuit à la température ambiante, essore le chlorhydrate d'imino-éther précipité, le lave avec 50 $cm^3$ d'éther anhydre et le sèche à l'air.

On recueille finalement 10,3 g (rendement : 86 %) de chlorhydrate de l'imino-éther éthylique de l'acide (méthyl-5 cyclopropyl-2 phénoxy)acétique sous forme d'un solide blanc non hygroscopique.

*d) Chlorhydrate de la (méthyl-5' cyclopropyl-2')phényloxyméthyl-2$\Delta_2$-imidazoline :*

Dans un ballon de 250 $cm^3$ à 3 tubulures muni d'un réfrigérant surmonté d'un tube de garde rempli de chlorure de calcium, d'un agitateur, d'une ampoule à brome, d'un thermomètre plongeant et d'un chandelier, on introduit sous atmosphère d'azote, 10,3 g (0,038 mol/g) de chlorhydrate de l'imino-éther éthylique de l'acide (méthyl-5 cyclopropyl-2 phénoxy)acétique et 40 $cm^3$ d'éthanol anhydre. Par l'ampoule à brome, on verse 2,2 $cm^3$ d'éthylène diamine, préalablement distillée sur de la potasse, puis séchée sur du siliporite, en agitant le mélange vigoureusement. On le porte ensuite pendant 6 h à la température du reflux. On filtre un insoluble que l'on lave par 100 $cm^3$ d'alcool anhydre, évapore le solvant du filtrat, triture le résidu par 50 $cm^3$ d'acétone préalablement distillée sur de la potasse, puis séchée sur du siliporite, essore le précipité et le lave avec 75 $cm^3$ d'acétone anhydre.

On recueille finalement 4,6 g (rendement : 45 %) de chlorhydrate de (méthyl-5' cyclopropyl-2')phénoxyméthyl-2$\Delta_2$-imidazoline qui, recristallisés dans 30 fois leur poids d'isopropanol, se présentent sous forme d'une substance blanche, peu soluble dans l'eau, qui fond à 212 - 218°C.

**Exemple 2**

**Chlorhydrate de (chloro-5' cyclopropyl-2')phénoxyméthyl-2$\Delta_2$-imidazoline R = Cl** N.D. 83 204

a) *chloro-5 cyclopropyl-2 phénol*

Préparé suivant le mode opératoire décrit pour le méthyl-5 cyclopropyl-2 phénol de l'exemple 1, à partir de 31,5 g (0,188 mol/g) de chloro-5 cyclopropyl-2 aniline avec un rendement de 32 %.

Pt Eb = 69 - 76°C/0,1 mm de mercure. PF : 30°C.

b) *chloro-5 cyclopropyl-2 phénoxyacétonitrile*

Préparé suivant le mode opératoire décrit dans l'exemple 1b) à partir de 6 g (0,0356 mol/g) de chloro-5 cyclopropyl-2 phénol avec un rendement brut de 95 %. On utilise le produit brut dans la réaction suivante :

c) *chlorhydrate de l'imino-éther éthylique de l'acide chloro-5 cyclopropyl-2 phénoxy acétique* :

A partir de 7 g du nitrile brut précédent, on recueille suivant le mode opératoire décrit dans l'exemple 1c), 9 g (rendement : 92 %) du chlorhydrate d'imino-éther.

d) *chlorhydrate de la (chloro-5'-cyclopropyl-2')phénoxyméthyl-2$\Delta_2$-imidazoline*

On fait réagir suivant le mode opératoire décrit dans l'exemple 1d) 9 g (0,031 mol/g) de chlorhydrate d'imino-éther obtenu ci-dessus avec 1,7 g (0,0282 mol/g) d'éthylène diamine anhydre dans 35 $cm^3$ d'alcool absolu. On chauffe à la température du reflux pendant 6 h, évapore l'alcool, triture le résidu dans 30 $cm^3$ d'eau, essore, lave le précipité successivement par 40 $cm^3$ puis trois fois 20 $cm^3$ d'acétone, le sèche à l'air et le recristallise finalement dans le méthanol.

On recueille ainsi 3,2 g (rendement : 36 %) de chlorhydrate de (chloro-5' cyclopropyl-2')phénoxyméthyl-2$\Delta_2$-imidazoline sous forme d'un composé blanc, peu soluble dans l'eau, qui fond à 248 - 250°C.

**Exemple 3 :**

**Chlorhydrate de (méthyl-5' cyclopropyl-2')phénoxyméthyl-2$\Delta_2$-imidazoline (I) R = $CH_3$** (N.D. 83 201)

a) *méthyl-5 cyclopropyl-2 phénoxyacétate d'éthyle*

Dans un ballon de 100 $cm^3$ à 3 tubulures muni d'un réfrigérant surmonté d'un tube de garde rempli de chlorure de calcium, d'un thermomètre plongeant et d'un agitateur, on introduit 6,5 g (0,044 mol/g) de méthyl-5 cyclopropyl-2 phénol, 35 $cm^3$ de méthyléthylcétone anhydre, 6,03 g (0,044 mol/g) de carbonate de potassium sec et 7,66 g (0,063 mol/g) de chloracétate d'éthyle. On chauffe ce mélange en l'agitant pendant 28 h à la température du reflux. On le refroidit, filtre les sels minéraux, les lave avec 60 $cm^3$ de méthyléthylcétone, évapore le solvant du filtrat, dissout le résidu dans

50 $cm^3$ d'éther, lave la solution éthérée deux fois successivement par 25 $cm^3$ de soude normale, extrait les eaux de lavage alcalines par deux fois successivement 50 $cm^3$ d'éther, réunit les extraits éthérés, les lave par 2 fois 50 $cm^3$ d'eau, les sèche sur du sulfate de sodium anhydre, filtre, évapore le solvant du filtrat et rectifie le résidu.

On recueille 7,4 g (rendement = 73 %) de méthyl-5 cyclopropyl-2 phénoxy-acétate d'éthyle sous forme d'un liquide incolore passant à 112°C sous 0,5 mm de mercure.

b) *méthyl-5 cyclopropyl-2 phénoxy N-(ß-aminoéthyl)acétamide*

Dans un ballon de 50 $cm^3$ à 3 tubulures muni d'un réfrigérant surmonté d'un tube de garde rempli de chlorure de calcium et d'un thermomètre plongeant, on introduit 5,3 g (0,0316 mol/g) de méthyl-5 cyclopropyl-2 phénoxyacétate d'éthyle et 7,6 g (0,126 mol/g) d'éthylène diamine anhydre. On chauffe ce mélange pendant 17 h à 100°C, on refroidit, on ajoute 20 $cm^3$ d'eau, on filtre un léger insoluble et on évapore l'eau et l'excès d'éthylène diamine sous vide. On dissout le résidu solide dans 30 $cm^3$ d'isopropanol, on ajoute 5 $cm^3$ d'une solution chlorhydrique d'isopropanol titrant 5,18 N, on glace la solution, on essore le chlorhydrate précipité, le lave par 20 $cm^3$ d'isopropanol, le sèche et le recristallise finalement dans 80 $cm^3$ d'isopropanol. On recueille 3,5 g (rendement = 40 %) de chlorhydrate de méthyl-5 cyclopropyl-2 phénoxy N-(ß-aminoéthyl) acétamide, sous forme d'un composé cristallisé blanc qui fond à 165 - 167°C.

c) *chlorhydrate de (méthyl-5' cyclopropyl-2')phénoxyméthyl-2$\Delta_2$* imidazoline

A 1,4 g de chlorhydrate de (méthyl-5 cyclopropyl-2) N-(ß-aminoéthyl)acétamide, on ajoute 50 $cm^3$ d'eau, 50 $cm^3$ d'éther et une solution saturée de carbonate de sodium jusqu'à pH 9. On décante la phase aqueuse, on évapore le solvant de la phase éthérée et on chauffe les 0,9 g de base solide mélangée à 0,2 g de chaux vive pendant 1 h à 200°C. On laisse refroidir, ajoute 5 $cm^3$ d'alcool isopropylique, filtre l'insoluble et acidifie le filtrat par quelques gouttes d'une solution chlorhydrique d'isopropanol. On essore le chlorhydrate d'imidazoline formé, le sèche et l'identifie par son P.F. et son spectre IR au même composé préparé selon l'exemple 1.

Les composés faisant l'objet de l'invention présentent d'intéressantes propriétés pharmacologiques, notamment vaso-constrictrices, susceptibles d'être mises à profit dans le traitement symptomatique des affections rhinologiques avec congestion nasale. 1) Le pouvoir vaso-constricteur des composés (I) faisant l'objet de l'invention a été évalué par mesure des réponses vaso-pressives engendrées par leur administration par voie intraveineuse chez le rat Wistar anesthésié et amyélé, selon la technique décrite par Shipley et Tilden (Proceedings of Society Experimental Biology, 1947, *64*, pages 453 - 455). Cette technique permet d'apprécier le pouvoir agoniste $\alpha$-adrénergique postsynaptique d'une substance. Le pouvoir vaso-constricteur des composés de l'invention a été comparé à ceux de la Cirazoline, l'agoniste $\alpha_1$-adrénergique postsynaptique le plus puissant et le plus spécifique connu à ce jour, et de la Fénoxazoline, également un agoniste $\alpha_1$-adrénergique postsynaptique actuellement commercialisé. Pour toutes ces substances, des courbes log doses cumulatives par voie intraveineuse/action hypertensive engendrée ont été établies sur des lots de 8 animaux anesthésiés, amyélés, bivagotomisés, atropinés et curarisés et leurs $DE_{50}$ (doses en µg par kg de base permettant d'engendrer une réponse égale à 50 % de la réponse maximale) ont été calculées et comparées entre elles.

Le tableau I rassemble les valeurs de ces $DE_{50}$. Il y apparaît que les composés ND 83201 et ND 83204 sont les agents vasoconstricteurs les plus actifs, leur puissance à cet égard étant environ 1,75 fois celle de la Cirazoline et 3 fois celle de la Fénoxazoline.

**Tableau I**

| Substances | R | DE 50 (µ/kg, I.V.) | Puissance relative vis-à-vis de la Cirazoline |
|---|---|---|---|
| CIRAZOLINE | H | 1,105 ± 0,132 | 1 |
| FENOXAZOLINE | | 1,790 ± 0,147 | 0,62 |
| ND 83 201 (I) | $CH_3$ | 0,630 ± 0,060 | 1,75 |
| ND 83 204 (I) | Cl | 0,644 ± 0,050 | 1,72 |

**2) Nature de l'effet agoniste $\alpha$-adrénergique postsynaptique vasoconstricteur du ND 83 201.**

Les expériences mentionnées ci-dessus ont été répétées, pour le ND 83 201, la Cirazoline et la Fénoxazoline, sur le même modèle expérimental, après administration préalable soit d'un antagoniste $\alpha_1$-adrénergique postsynaptique spécifique (prazosine, 20 µg/ kg/min/5 min), soit un antagoniste $\alpha_2$-adrénergique postsynaptique (yohimbine, 60 µg/kg/min/5 min).

Pour les trois substances, des courbes log doses cumulatives par voie intraveineuse/action hypertensive engendrée ont été tracées et les $DE_{50}$ en µg par kg de base ont été calculées avant et après administration des antagonistes, sur des lots de 8 animaux.

Le tableau II montre les résultats obtenus. Il y apparaît clairement que l'effet vaso-constricteur du ND 83 201 :

- est très puissamment antagonisé par la prazosine et même légèrement plus que ceux de la Cirazoline et de la Fénoxazoline,

– n'est pratiquement pas antagonisé par la yohimbine, tout comme ceux de la Cirazoline et de la Fénoxazoline.

Ces résultats montrent donc clairement que le ND 83 201 est un antagoniste $\alpha_1$-adrénergique postsynaptique spécifique.

**Tableau II**

| Substances | DE 50 (µg/kg,I.V) | DE 50 (µg/kg,I.V) après prazosine | $DE_{50}$ après / $DE_{50}$ avant prazosine | DE 50 (µ/kg,I.V) après yohimbine | DE 50 après / $DE_{50}$ avant yohimbine |
|---|---|---|---|---|---|
| Cirazoline | 1,105 ± 0,132 | 8,568 ± 0,721 | 7,75 | 1,180 ± 0,108 | 1,05 |
| Fénoxazoline | 1,790 ± 0,147 | 16,205 ± 1,240 | 9,05 | 1,804 ± 0,237 | 1,01 |
| ND 83 201 | 0,630 ± 0,060 | 7,284 ± 0,974 | 11,56 | 0,805 ± 0,076 | 1,28 |

**3) Autres propriétés pharmacologiques**

a) Le ND 83 201 est une substance sympathomimétique directe. En effet, comme pour la Cirazoline et la Fénoxazoline, les effets vaso-constricteurs du ND 83 201 ne sont pas, sauf aux très fortes doses, modifiés par un prétraitement des animaux à la réserpine, le modèle expérimental étant le même que celui utilisé dans les expériences précédentes.

b) le ND 83 201 ne possède pas d'activité agoniste $\alpha_2$-adrénergique présynaptique.

La technique utilisée est celle du rat Wistar anesthésié, amyélé, bivagotomisé, atropiné et curarisé, et dont on stimule électriquement la moelle (Gillespie et Muir - British Journal of Pharmacology and Chemotherapy, 1967, *30*, pages 78 - 87) de manière sélective au niveau de ses émergences cardiaques afin d'engendrer une tachicardie supramaximale. Sur ce modèle expérimental, les agonistes $\alpha_2$-adrénergiques présynaptiques, comme la clonidine, réduisent cette tachycardie.

Le ND 83 201 et la Cirazoline ont donc été étudiés sur ce modèle expérimental comparativement à la clonidine sur des lots de 8 animaux. Alors que la clonidine a réduit la tachycardie submaximale (effet antagonisé par la yohimbine), ni le ND 83 201, ni la Cirazoline n'ont engendré le moindre effet, démontrant ainsi qu'ils étaient dénués de toute propriété agoniste $\alpha_2$-adrénergique présynaptique.

c) le ND 83 201 ne possède pas d'activité antagoniste $\alpha_2$-adrénergique présynaptique.

La technique utilisée est celle du rat Wistar anesthésié, amyélé, bivagotomisé, atropiné et curarisé dont on stimule la moelle (Gillespie et Muir - British Journal of Pharmacology and Chemotherapy, 1967, *30*, pages 78 - 87) comme décrit ci-dessus pour engendrer une tachycardie supramaximale. On réduit cette tachycardie par administration d'un agoniste $\alpha_2$-adrénergique présynaptique, la clonidine (40 µg/kg, I.V.). Sur ce modèle expérimental, les antagonistes $\alpha_2$-adrénergiques présynaptiques comme la phentolamine, mais non pas les antagonistes $\alpha_1$-adrénergiques, comme la prazosine, rétablissent la tachycardie supramaximale.

Le ND 83 201 et la Cirazoline ont donc été étudiés sur ce modèle expérimental comparativement à la phentolamine et à la prazosine sur des lots de 8 animaux

Le tableau III montre les résultats obtenus. Alors que la phentolamine rétablit la tachycardie supramaximale, la prazosine, le ND 83 201 et la Cirazoline sont sans action. Ceci démontre que ces deux dernières substances n'exercent, comme la prazosine, aucun effet antagoniste $\alpha_2$-adrénergique présynaptique.

**Tableau III**

| | FREQUENCE CARDIAQUE (batt/min) | | | |
|---|---|---|---|---|
| Substance étudiée (µg/kg, I.V.) | Avant stimulation électrique médullaire | Sous stimulation électrique (tachycardie supramaximale) | Sous stimulation électrique et après clonidine (40µ/kg, I.V) | Sous stimulation électrique après clonidine et substance étudiée |
| Phentolamine (700) * | 355,0 ± 10,1 | 455,6 ± 11,5 | 411,2 ± 8,9 | 452,5 ± 10,6 |
| Prazosine (700) * | 338,7 ± 6,3 | 440,0 ± 9,6 | 391,9 ± 8,2 | 388,1 ± 7,0 |
| Cirazoline (2,5) * | 360,7 ± 13,1 | 452,1 ± 10,5 | 395,7 ± 14,1 | 403,6 ± 14,8 |
| ND 83 201 (18,5)* | 350,0 ± 4,0 | 448,7 ± 7,7 | 391,2 ± 5,9 | 403,1 ± 6,0 |

*Les doses indiquées ici sont celles qui ont engendré l'effet maximal.

d) Le ND 83 201 ne possède pas d'activité agoniste ß-adrénergique postsynaptique. En effet, sur le même modèle expérimental que celui utilisé dans les expériences précédentes, la $DE_{50}$ du ND 83 201 n'est pas modifiée par un prétraitement par un antagoniste ß-adrénergique, le propanolol (150 µg/kg/min/5 min).

**Revendications** pour les états contractants: BE, CH, DE, FR, GB, IT, LI, LU, NL, SE

1. Nouvelles imidazolines de formule générale (I)

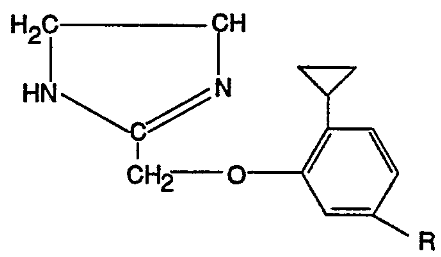

(I)

ainsi que les sels d'addition formés par ces bases avec les acides minéraux ou organiques pharmaceutiquement acceptables, éventuellement doués de propriétés pharmacologiques propres, caractérisées en ce que le radical R est un radical méthyle ou un atome de chlore.

2. Composés selon la revendication 1, dans lesquels les acides qui forment des sels d'addition avec les bases (I) sont soit des acides minéraux tels l'acide chlorhydrique, l'acide sulfurique ou l'acide nitrique, soit des acides organiques tels l'acide acétique, l'acide tartrique ou l'acide citrique.

3. Composés selon la revendication 1, dans lesquels les acides qui forment des sels d'addition avec les bases (I) sont doués d'une activité thérapeutique complémentaire à celle des composés (I) tels les acides suivants : acide prednisolone benzoyl métasulfonique-21, l'acide fluoro-9α méthyl-16ß trihydroxy-11ß,17α,21 prégnadiène1,4 dione-3,20 orthophosphorique-21, l'acide trihydroxy-11ß,17α, 21 prégnène-4 dione-3,20 orthophosphorique-21 ; l'acide trihydroxy-11ß,17α,21 prégnène-4 dione-3,20 succinique-21.

4. Procédé de préparation des composés (I) selon lequel on fait réagir l'éthylène diamine avec un imino-éther de formule générale (II)

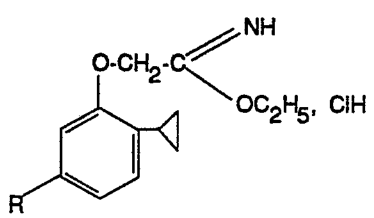

(II)

R ayant la même signification que dans la revendication 1.

5. Procédé suivant la revendication 4, caractérisé en ce que la condensation s'effectue au sein d'un alcool de faible poids moléculaire, de préférence d'alcool éthylique, et à la température du reflux de ce solvant, la durée de ce chauffage étant de quelques heures, de préférence quatre à six heures.

6. Procédé de préparation des composés (I), caractérisé en ce qu'on chauffe une amido-amine de formule générale (V), dans laquelle R a la même signification que dans la revendication 1, à une température de 150 - 200°C en présence de chaux vive.

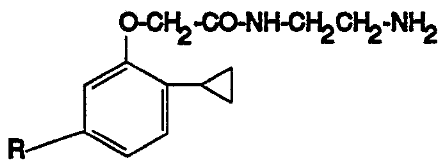

(V)

7. Composés de formule (I) pour l'utilisation vasoconstrictrice et décongestionnante nasale et pour le traitement symptomatique de divers syndromes rhinologiques.

8. Médicaments pour l'utilisation en thérapeutique comme vasoconstricteurs et décongestionnants pour le nez et pour le traitement symptomatique de syndromes rhinologiques, caractérisés en ce qu'ils contiennent un composé de formule générale (I) et éventuellement tout excipient approprié à leur mise en forme pharmaceutique et, éventuellement, d'autres substances médicamenteuses actives avec lesquelles lesdits composés de formule générale (I) et leurs sels d'addition sont compatibles.

**Revendications** pour l'état contractant: AT

1. Procédé de préparation de nouvelles imidazolines de formule générale (I) :

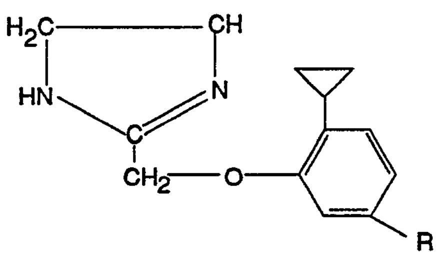

(I)

dans laquelle R représente un radical méthyle ou un atome de chlore ainsi que les sels d'addition formés par ces bases avec les acides minéraux ou organiques pharmaceutiquement acceptables, éventuellement doués de propriétés pharmacologiques propres, caractérisé en ce qu'on fait réagir l'éthylène diamine avec un imino-éther de formule générale (II):

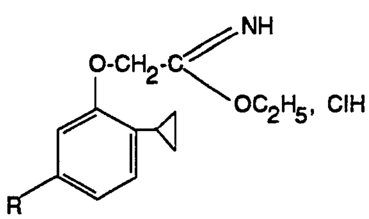

(II)

R ayant la signification mentionnée ci-dessus.

2. Procédé suivant la revendication 1, caractérisé en ce que la condensation s'effectue au sein d'un alcool de faible poids moléculaire, de préférence d'alcool éthylique, et à la température du reflux de ce solvant, la durée de ce chauffage étant de quelques heures, de préférence quatre à six heures.

3. Une variante du procédé selon la revendication 1 ci-dessus selon laquelle on chauffe une amido-amine de formule générale (V), dans laquelle R a la même signification que dans la revendication 1, à une température de 150 - 200°C en présence de chaux vive.

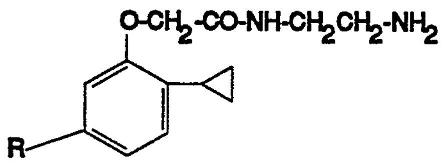

(V)

**Patentansprüche** für die Vertragsstaaten BE, CH, DE, FR, GB, IT, LI, LU, NL, SE:

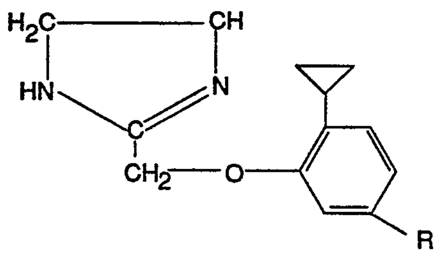

(I)

1. Neue Imidazoline der allgemeinen Formel (I) sowie die Additionssalze, die aus diesen Basen mit pharmazeutisch akzeptablen Mineral- oder organischen Säuren, die gegebenenfalls mit besonderen pharmakologischen Eigenschaften ausgestattet sind, gebildet sind, dadurch gekennzeichnet, daß der Rest R eine Methylgruppe oder ein Chloratom ist.

2. Verbindungen nach Anspruch 1, in denen die mit den Basen (I) Additionssalze bildenden Säuren entweder Mineralsäuren, wie Salzsäure, Schwefelsäure oder Salpetersäure, oder organische Säuren, wie Essigsäure, Weinsäure oder Zitronensäure, sind.

3. Verbindungen nach Anspruch 1, in denen die mit den Basen (I) Additionssalze bildenden Säuren mit einer zu jener der Verbindungen (I) komplementären therapeutischen Aktivität ausgestattet sind, wie die folgenden Säuren: Prednisolonbenzoyl-21-metasulfonsäure, 9α-Fluor-16ß-methyl-11ß,17α,21-trihydroxy1,4-pregnadien-3,20-dion-21-orthophosphorsäure; 11ß,17α,21-Trihydroxy-4-pregnen-3,20-dion-21-orthophosphorsäure; 11ß,17α,21-Trihydroxy-4-pregnen-3,20-dion-21-bernsteinsäure.

4. Verfahren zur Herstellung der Verbindungen (I), bei welchem man Ethylendiamin mit einem Iminoether der allgemei-

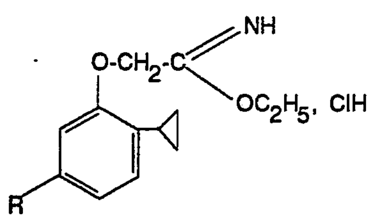

(II)

nen Formel (II) wobei R dieselbe Bedeutung wie in Anspruch 1 hat, reagieren läßt.

5. Verfahren nach Anspruch 4, dadurch gekennzeichnet, daß die Kondensation in einem Alkohol mit niedrigem Molekulargewicht, vorzugsweise Ethylalkohol, und bei der Rückflußtemperatur dieses Lösungsmittels erfolgt, wobei die Dauer dieser Erhitzung einige Stunden, vorzugsweise vier bis sechs Stunden, beträgt.

6. Verfahren zur Herstellung der Verbindungen (I), dadurch gekennzeichnet, daß man ein Amidoamin der allgemeinen

Formel (V), worin R dieselbe Bedeutung wie in Anspruch 1 hat, in Gegenwart von Ätzkalk auf eine Temperatur von

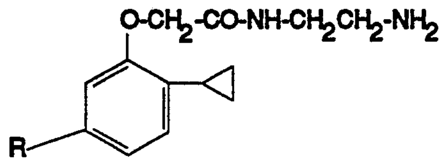

(V)

150 - 200°C erhitzt.

7. Verbindungen der Formel (I) zur gefäßkontrahierenden und kongestionsvermindernden Anwendung bei der Nase und zur symptomatischen Behandlung verschiedener rhinologischer Syndrome.

8. Medikamente zur therapeutischen Anwendung als Vasokonstriktoren und Dekongestionsmittel für die Nase und zur symptomatischen Behandlung von rhinologischen Syndromen, dadurch gekennzeichnet, daß sie eine Verbindung der allgemeinen Formel (I) und gegebenenfalls jeden zur Bildung ihrer pharmazeutischen Form geeigneten Exzipienten und gegebenenfalls weitere aktive medikamentöse Substanzen, mit denen die Verbindungen der allgemeinen Formel (I) und ihre Additionssalze kompatibel sind, enthalten.

**Patentansprüche** für den Vertragsstaat AT:

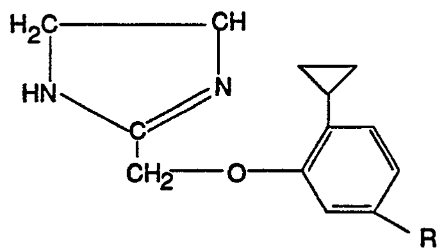

(I)

1. Verfahren zur Herstellung neuer Imidazoline der allgemeinen Formel (I) worin R eine Methylgruppe oder ein Chloratom darstellt, sowie der Additionssalze, die aus diesen Basen mit pharmazeutisch akzeptablen Mineral- oder organischen Säuren, die gegebenenfalls mit besonderen pharmakologischen Eigenschaften ausgestattet sind, gebildet sind, dadurch gekennzeichnet, daß man Ethylendiamin mit einem Iminoether der

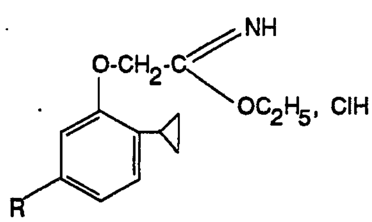

(II)

allgemeinen Formel (II) wobei R die oben angegebene Bedeutung hat, reagieren läßt.

2. Verfahren nach Anspruch 1, dadurch gekennzeichnet, daß die Kondensation in einem Alkohol mit niedrigem Molekulargewicht, vorzugsweise Ethylalkohol, und bei der Rückflußtemperatur dieses Lösungsmittels erfolgt, wobei die Dauer dieser Erhitzung einige Stunden, vorzugsweise vier bis sechs Stunden, beträgt.

3. Variante des Verfahrens nach dem obigen Anspruch 1, gemäß welcher man ein Amidoamin der allgemeinen Formel (V), worin R dieselbe Bedeutung wie in Anspruch 1 hat, in Gegenwart von Ätzkalk auf eine Temperatur von 150 - 200°C erhitzt.

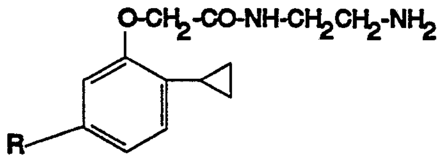

(V)

Claims for contracting States : BE, CH, DE, FR, GB, IT, LI, LU, NL, SE

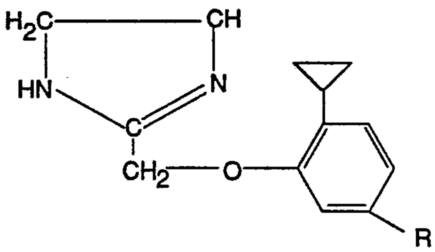

(I)

1. New imidazolines of general formula (I) and the addition salts formed by these bases with the pharmaceutically acceptable organic or mineral acids, optionally having their own pharmacological properties, characterized in that the radical R is methyl or an atom of chloride.

2. Compounds according to claim 1, in which the acids forming addition salts with the bases (I) are either mineral acids such as hydrochloric acid, sulfuric acid, or nitric acid, or organic acids such as acetic acid, tartric acid or citric acid.

3. Compounds according to claim 1, in which the acids forming addition salts with the bases (I) have a therapeutical activity which is complementary to that of compounds (I) such as the following acids : the prednisolone-21-benzoyl-metasulphonic acid ; 9 alpha-fluoro-16beta-methyl-11beta, 17alpha, 21-trihydroxy-1,4-pregnadiene-3,20-dione-21-orthophosphoric acid ; 11beta, 17alpha,21-trihydroxy-1,4-pregnadiene-3,20-dione-21-orthophosphoric acid ; 11beta, 17alpha,21-trihydroxy-4-pregnene-3,20dione-21-orthophosphoric acid ; 11beta, 17alpha,21-trihydroxy-4-pregnene-3,20-dione-21-succinic acid.

4. Process of the preparation of compounds (I) according to which the ethylene diamine is reacted with an imino-ether

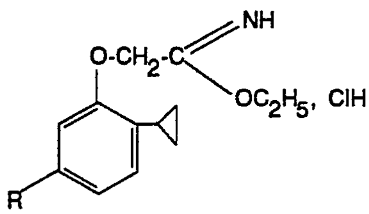

(II)

of general formula (II)
R being as indicated in claim 1.

5. Process according to claim 4, characterized in that the condensation in an alcohol of low molecular weight, preferably ethyl alcohol at the solvent reflux temperature, the heating period being several hours, preferably four to six hours.

6. Process for the preparation of compounds (I), characterized in that an amido-amine of general formula (V), in which R is as indicated in claim 1, is heated at a temperature of 150 - 200°C in the presence of quick lime,

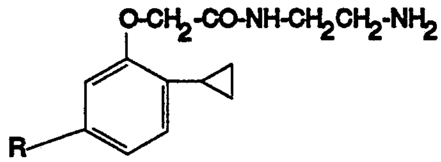

(V)

7. Compounds of formula (I) usable as vasoconstrictor and nasal congestion-relieving agent and for the symptomatic treatment of various rhinological syndromes.

8. Drugs for the therapeutic use as vasoconstrictors and nasal congestion-relieving agents, and for the symptomatic treatment of rhinological syndromes, characterized in that they contain a compound of general formula (I) and optionally any excipient needed for their pharmaceutical presentation and, optionally, other active medicinal substances with which said compounds of formula (I) and their addition salts are compatible.

**Claims** for Contracting State : AT

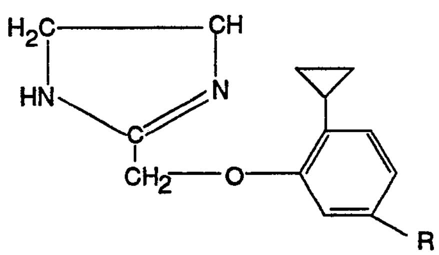

(I)

1. Process for the preparation of new imidazolines of general formula (I) :
in which R is a methyl radical or a chlorine atom and of the addition salts formed by these bases with the pharmaceutically acceptable mineral or organic acids, optionally having their own pharmacological properties, characterized in that

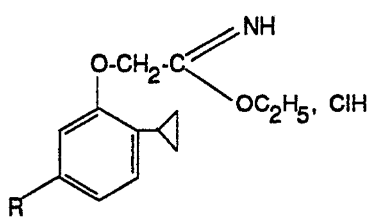

(II)

the diamine ethylene is reacted with an imino-ether of general formula (II) :
R being as indicated above.

2. Process according to claim 1, characterized in that the condensation takes place in a low molecular weight alcohol, preferably ethyl alcohol, and at the reflux temperature of that solvent, the heating period being several hours, preferably four to six hours.

3. A variant of the process according to claim 1, above, according to which an amido-amine of general formula (V) in which R is as indicated in claim 1 is heated to a temperature of 150 - 200°C in the presence of quick lime :

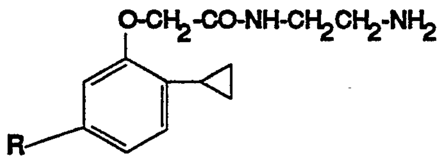

(V)